# EUROPEAN PATENT APPLICATION

(11) **EP 2 684 560 A1**
(43) Date of publication of application: **15.01.2014**
(21) Application number: 12802671.3
(22) Date of filing: 22.06.2012
(51) Int. Cl.: A61K 9/08, A61K 47/10, A61K 47/18, A61K 47/36

(54) **OPHTHALMIC SOLUTION CONTAINING HYALURONIC ACID OR SALT THEREOF AND PROPYLENE GLYCOL**

(30) Priority: 23.06.2011 JP 2011139566
(71) Applicant: SANTEN PHARMACEUTICAL CO., LTD., Higashiyodogawa-ku, Osaka-shi, Osaka 533-8651 (JP)
(72) Inventor: INAGAKI, Koji, Ikoma-shi Nara 630-0101 (JP); OGAWA, Toshihiro, Ikoma-shi Nara 630-0101 (JP); HORIBE, Yoshihide, Ikoma-shi Nara 630-0101 (JP); DOTA, Atsuyoshi, Ikoma-shi Nara 630-0101 (JP); NAGANO, Takashi, Ikoma-shi Nara 630-0101 (JP); NAKAMURA, Masatsugu, Ikoma-shi Nara 630-0101 (JP); NAKADA, Yuichiro, Osaka-shi Osaka 533-8651 (JP)
(74) Representative: Müller-Boré & Partner Patentanwälte
(86) International application number: PCT/JP2012/065957
(87) International publication number: WO 2012/176865

(57) **Abstract**

An aqueous ophthalmic solution containing hyaluronic acid or a salt thereof at a concentration from 0.03 to 0.5% (w/v) and propylene glycol at a concentration from 0.1 to 1.0% (w/v) comprises as a sole preservative, benzalkonium chloride at a concentration from 0.001 to 0.002% (w/v) and comprises an ionic tonicity agent at such a concentration that an osmotic pressure ratio of the ophthalmic solution becomes from 0.9 to 1.1.

## Description

### TECHNICAL FIELD

The present invention relates to an aqueous ophthalmic solution containing hyaluronic acid or a salt thereof at a concentration from 0.03 to 0.5% (w/v) and propylene glycol at a concentration from 0.1 to 1.0% (w/v), which comprises as a sole preservative, benzalkonium chloride at a concentration from 0.001 to 0.002% (w/v) and comprises an ionic tonicity agent at such a concentration that an osmotic pressure ratio of the ophthalmic solution becomes from 0.9 to 1.1. In addition, the present invention also relates to a method of improving preservative effectiveness of an ophthalmic solution containing sodium hyaluronate at a concentration of 0.1 or 0.3% (w/v) and benzalkonium chloride as a sole preservative, setting kinematic viscosity of the ophthalmic solution to 3.0 to 4.0 or 17 to 30 mm²/s, and preventing drip of the ophthalmic solution, which comprises a step (a) of mixing sodium hyaluronate in such an amount that a concentration in the ophthalmic solution becomes from 0.1 or 0.3% (w/v), benzalkonium chloride in such an amount that a concentration in the ophthalmic solution becomes from 0.001 to 0.002% (w/v), propylene glycol in such an amount that a concentration in the ophthalmic solution becomes from 0.1 to 1.0% (w/v), and an ionic tonicity agent in such an amount that an osmotic pressure ratio of the ophthalmic solution becomes from 0.9 to 1.1, a step (b) of adjusting pH of the ophthalmic solution to 6.0 to 7.0, and a step (c) of filling an eye drop container with the ophthalmic solution.

### BACKGROUND ART

Dry eye is a disease starting from such a minor symptom as dryness of an eye or uncomfortable feeling as if something were in an eye, and aggravation thereof significantly interferes with daily life. The number of patients suffering from dry eye has increased year by year with emergence of the aging society or increase in works involved with video display terminals (VDT) such as a personal computer, and the number of estimated patients is allegedly 10 million or more in the United States and 8 million or more also in Japan.

Though morbidity of dry eye has not completely been clarified, it has been known that dry eye causes keratoconjunctive epithelium disorder and ultimately causes visual impairments. Therefore, it is extremely important to treat appropriately in an early stage, keratoconjunctive epithelium disorder caused by dry eye.

Ocular instillation is currently most common as a method of treatment of dry eye, and in Japan, an ophthalmic solution containing sodium hyaluronate is widely used for dry eye treatment.

As an ophthalmic solution containing sodium hyaluronate (hereinafter also simply referred to as a "sodium hyaluronate ophthalmic solution"), for the purpose of use thereof multiple times, a multiple-dose type allowing free opening and re-sealing of a cap or the like (Hyalein® ophthalmic solution 0.1%, Hyalein® ophthalmic solution 0.3%, and the like) and a unit-dose type intended for single dose (Hyalein® mini ophthalmic solution 0.1%, Hyalein® mini ophthalmic solution 0.3%, and the like) are available.

With regard to the multiple-dose type sodium hyaluronate ophthalmic solution, a cap is opened and re-sealed, and therefore benzalkonium chloride is added thereto as a preservative. On the other hand, since the unit-dose type is intended for single dose (disposable), no preservative is added.

It has been pointed out that such a preservative as benzalkonium chloride has the possibility of inducing corneal damage. For example, as disclosed in Clinical and Experimental Ophthalmology, 32, 180-184 (2004) (NPD 1), benzalkonium chloride has been known to have the possibility of causing corneal epithelium disorder in a concentration-dependent manner. As described previously, since dry eye is essentially a disease accompanying corneal epithelium disorder, a unit-dose type sodium hyaluronate ophthalmic solution is used for severe dry eye patients. On the other hand, in light of a problem of production cost and the like, it is difficult to prescribe a unit-dose type sodium hyaluronate ophthalmic solution to all dry eye patients. Therefore, it is also possible that incidence of corneal epithelium disorder is lowered by lowering a concentration of a preservative in a multiple-dose type ophthalmic solution. Actually, however, if a concentration of a preservative in the eye drop is lowered, preservative effectiveness sufficient for use as the multiple-dose type will not be obtained. In addition, there is also a concern that, due to change in blended ingredients accompanying decrease in preservative, physiochemical properties of the ophthalmic solution differ from those of a conventional sodium hyaluronate ophthalmic solution.

Though Japanese Patent Laying-Open No. 2004-359629 (PTD 1), Japanese Patent Laying-Open No. 2009-196903 (PTD 2), and Japanese Patent Laying-Open No. 2009-161454 (PTD 3) disclose an ophthalmic solution containing sodium hyaluronate, benzalkonium chloride, and propylene glycol, they are completely silent about an ophthalmic solution having sufficient preservative effectiveness and having physiochemical properties the same as those of the conventional sodium hyaluronate ophthalmic solution while a concentration of benzalkonium chloride in the ophthalmic solution is equal to or lower than 0.0025%.

### CITATION LIST

### PATENT DOCUMENT

PTD 1: Japanese Patent Laying-Open No. 2004-359629
PTD 2: Japanese Patent Laying-Open No. 2009-196903
PTD 3: Japanese Patent Laying-Open No. 2009-161454

### NON PATENT DOCUMENT

NPD 1: Clinical and Experimental Ophthalmology, 32, 180-184 (2004)

### SUMMARY OF INVENTION

### TECHNICAL PROBLEM

As above, it is an interesting task to search for an ophthalmic solution having sufficient preservative effectiveness and having physiochemical properties the same as those of the conventional sodium hyaluronate ophthalmic solution while a concentration of benzalkonium chloride is lowered.

### SOLUTION TO PROBLEM

The present inventors have found that a sodium hyaluronate ophthalmic solution to which 0.002% (w/v) benzalkonium chloride has been added does not achieve sufficient preservative effectiveness, whereas addition of propylene glycol thereto will achieve sufficient preservative effectiveness even when a concentration of benzalkonium chloride is lowered to 0.001 % (w/v).

On the other hand, the present inventors have found that if more than 1% (w/v) propylene glycol is added to a sodium hyaluronate ophthalmic solution, an allowable range of kinematic viscosity and/or osmotic pressure ratio of the sodium hyaluronate ophthalmic solution described in "Hyalein® ophthalmic solution 0.1%, Hyalein® ophthalmic solution 0.3%, Hyalein® mini ophthalmic solution 0.1%, and Hyalein® mini ophthalmic solution 0.3% medical supply interview form, November 2010 (Revised Seventh Edition) (hereinafter also referred to as 'Hyalein interview form')" is exceeded, and have completed the present invention in which the upper limit of an amount of addition of propylene glycol is set to 1% (w/v).

In addition, the present inventors have also found that a propylene glycol-containing sodium hyaluronate ophthalmic solution is less likely to drip and it also has an effect to protect corneal epithelial cells against drying, as will be described later.

Namely, the present invention is directed to an aqueous ophthalmic solution containing hyaluronic acid or a salt thereof at a concentration from 0.03 to 0.5% (w/v) and propylene glycol at a concentration from 0.1 to 1.0% (w/v), which comprises as a sole preservative, benzalkonium chloride at a concentration from 0.001 to 0.002% (w/v) and comprises an ionic tonicity agent at such a concentration that osmotic pressure ratio of the ophthalmic solution becomes from 0.9 to 1.1 (hereinafter also simply referred to as the "present ophthalmic solution").

In addition, another embodiment of the present invention is directed to the present ophthalmic solution comprising a buffer and a pH adjuster in such an amount that pH becomes from 6.0 to 7.0.

In addition, another embodiment of the present invention is directed to the present ophthalmic solution comprising an edetate at a concentration from 0.001 to 0.1% (w/v).

In addition, another embodiment of the present invention is directed to the present ophthalmic solution in which a concentration of hyaluronic acid or a salt thereof is from 0.1 to 0.3% (w/v).

In addition, another embodiment of the present invention is directed to the present ophthalmic solution in which a concentration of propylene glycol is from 0.25 to 0.75% (w/v).

In addition, another embodiment of the present invention is directed to the present ophthalmic solution in which a concentration of benzalkonium chloride is from 0.001 to 0.0015% (w/v).

In addition, another embodiment of the present invention is directed to an aqueous ophthalmic solution consisting essentially of hyaluronic acid or a salt thereof at a concentration from 0.03 to 0.5% (w/v), propylene glycol at a concentration from 0.1 to 1.0% (w/v), benzalkonium chloride at a concentration from 0.001 to 0.002% (w/v), an ionic tonicity agent at such a concentration that osmotic pressure ratio of the ophthalmic solution becomes from 0.9 to 1.1, a buffer and a pH adjuster in such an amount that pH of the ophthalmic solution becomes from 6.0 to 7.0, and an edetate at a concentration from 0.001 to 0.1 % (w/v).

In addition, another embodiment of the present invention is directed to an aqueous ophthalmic solution consisting essentially of hyaluronic acid or a salt thereof at a concentration from 0.1 to 0.3% (w/v), propylene glycol at a concentration from 0.25 to 0.75% (w/v), benzalkonium chloride at a concentration from 0.001 to 0.0015% (w/v), an ionic tonicity agent at such a concentration that osmotic pressure ratio of the ophthalmic solution becomes from 0.9 to 1.1, a buffer and a pH adjuster in such an amount that pH of the ophthalmic solution becomes from 6.0 to 7.0, and an edetate at a concentration from 0.001 to 0.1% (w/v).

In addition, the present invention is directed to a method of improving preservative effectiveness of an aqueous ophthalmic solution containing sodium hyaluronate at a concentration of 0.1 or 0.3% (w/v) and benzalkonium chloride as a sole preservative, setting kinematic viscosity of the ophthalmic solution to 3.0 to 4.0 or 17 to 30 mm²/s, and preventing drip of the ophthalmic solution, comprising a step (a) of mixing sodium hyaluronate in such an amount that concentration in the ophthalmic solution becomes from 0.1 or 0.3% (w/v), benzalkonium chloride in such an amount that a concentration in the ophthalmic solution becomes to 0.001 to 0.002% (w/v), propylene glycol in such an amount that a concentration in the ophthalmic solution becomes from 0.1 to 1.0% (w/v), and an ionic tonicity agent in such an amount that osmotic pressure ratio of the ophthalmic solution becomes from 0.9 to 1.1, a step (b) of adjusting pH of the ophthalmic solution to 6.0 to 7.0, and a step (c) of filling an eye drop container with the ophthalmic solution (hereinafter, also simply collectively referred to as the "present method").

In addition, in the step (a) of the present method, an edetate in such an amount that a concentration in the ophthalmic solution becomes from 0.001 to 0.1 % (w/v) is preferably further mixed.

In addition, in the step (a) of the present method, propylene glycol in such an amount that a concentration in the ophthalmic solution becomes from 0.25 to 0.75% (w/v) is preferably mixed.

In addition, in the step (a) of the present method, benzalkonium chloride in such an amount that a concentration in the ophthalmic solution becomes from 0.001 to 0.0015% (w/v) is preferably mixed.

### ADVANTAGEOUS EFFECTS OF INVENTION

The present ophthalmic solution achieves sufficient preservative effectiveness by comprising propylene glycol despite the fact that a concentration of benzalkonium chloride in the ophthalmic solution is equal to or lower than 0.002% (w/v), while it has kinematic viscosity and osmotic pressure ratio allowed as the ophthalmic solution containing hyaluronic acid or a salt thereof (hereinafter also simply referred to as a "hyaluronic acid ophthalmic solution "). In addition, as is clear from results in drip check tests and evaluation test of protective effect on corneal epithelial cell which will be described later, the present ophthalmic solution has also such an effect as less likeliness of drip and protection of corneal epithelial cells against drying.

### BRIEF DESCRIPTION OF DRAWINGS

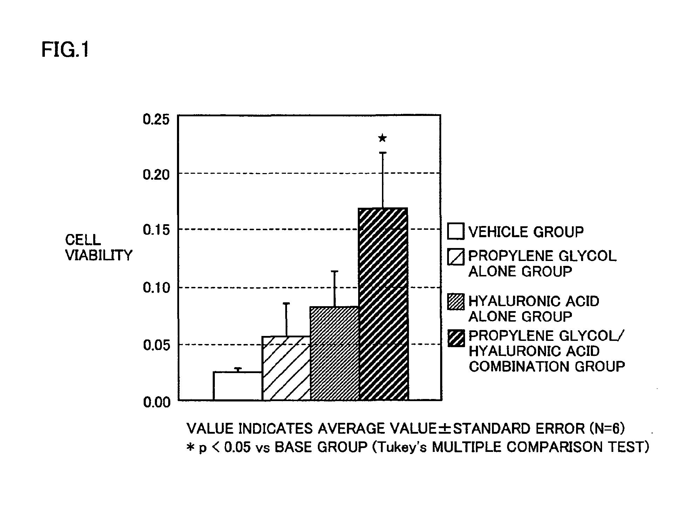

Fig. 1 is a graph showing influence by a propylene glycol-containing sodium hyaluronate ophthalmic solution on corneal epithelial cell damage due to drying load, in which the ordinate representing cell viability.

### DESCRIPTION OF EMBODIMENTS

The present ophthalmic solution is an aqueous ophthalmic solution containing hyaluronic acid or a salt thereof at a concentration from 0.03 to 0.5% (w/v) and propylene glycol at a concentration from 0.1 to 1.0% (w/v), characterized in that the ophthalmic solution comprises as a sole preservative, benzalkonium chloride at a concentration from 0.001 to 0.002% (w/v) and comprises an ionic tonicity agent at such a concentration that an osmotic pressure ratio of the ophthalmic solution becomes from 0.9 to 1.1. It is noted that the "aqueous ophthalmic solution" in the present invention refers to an ophthalmic solution comprising water as a vehicle.

The present ophthalmic solution can be used for treatment not only of keratoconjunctive epithelium disorder accompanying such endogenous conditions as dry eye (xerophthalmia syndrome), Sjögren syndrome, and Stevens-Johnson syndrome but also of keratoconjunctive epithelium disorder accompanying exogenous conditions, which is post-operative or drug-induced, or caused by trauma or use of contact lenses, or the like. Such a present ophthalmic solution achieves sufficient preservative effectiveness by containing propylene glycol despite the fact that a concentration of benzalkonium chloride in the ophthalmic solution is equal to or lower than 0.002% (w/v), while it has kinematic viscosity and osmotic pressure ratio allowed as the hyaluronic acid ophthalmic solution. In addition, the present ophthalmic solution also has such an effect as less likeliness of drip and protection of corneal epithelial cells against drying.

The hyaluronic acid in the present invention is a compound shown in a general formula (1) below: 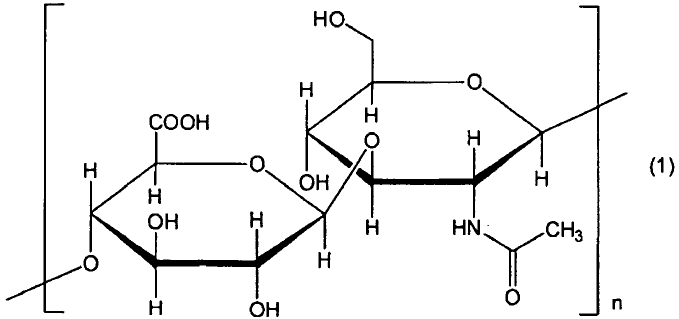 [where n represents a natural number].

Hyaluronic acid having an average molecular weight from 500,000 to 3,900,000 is preferred as "hyaluronic acid" in the present invention, and hyaluronic acid having an average molecular weight from 500,000 to 1,200,000 is further preferred.

Salt of hyaluronic acid is not particularly restricted, so long as it is a pharmaceutically acceptable salt, and examples thereof include: salt with such inorganic acids as hydrochloric acid, hydrobromic acid, hydriodic acid, nitric acid, sulfuric acid, and phosphoric acid; salt with such organic acids as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate ester, methyl sulfate, naphthalenesulfonic acid, and sulfosalicyclic acid; quaternary ammonium salt with methyl bromide, methyl iodide, and the like; salt with such halogen ions as bromine ions, chlorine ions, and iodine ions; salt with such an alkali metal as lithium, sodium, and potassium; salt with such an alkaline earth metal as calcium and magnesium; metal salt with iron, zinc, and the like; salt with ammonia; salt with such organic amines as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, and N,N-bis(phenylmethyl)-1,2-ethanediamine; and the like.

Sodium salt shown in a general formula (2) below (hereinafter also referred to as "sodium hyaluronate") is preferred as "salt of hyaluronic acid" in the present invention: 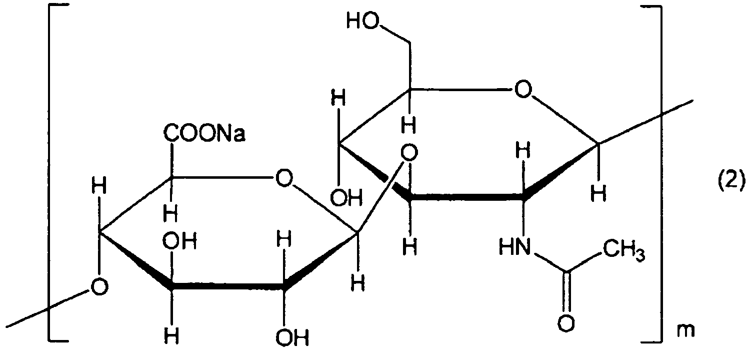 [where m represents a natural number].

"Hyaluronic acid or a salt thereof' in the present invention may be in a form of a hydrate or a solvate.

In a case where a geometric isomer or an optical isomer is present for hyaluronic acid, the isomer or salt thereof is also encompassed in the scope of the present invention. In addition, in a case where a proton tautomerism is present for hyaluronic acid, the tautomer or salt thereof is also encompassed in the present invention.

In a case where a polymorph and a polymorph group (a polymorphic system) are present in hyaluronic acid or a salt thereof, a hydrate, or a solvate, those polymorph and polymorph group (polymorphic system) are also encompassed in the present invention. Here, the polymorph group (polymorphic system) means an individual crystal form in each stage in a case where a crystal form changes depending on conditions and states for manufacturing, crystallization, storage, and the like of those crystals (the present state including also a formulated state) and the entire process thereof.

"Hyaluronic acid or a salt thereof' can also be manufactured in accordance with a method common in the field of synthetic organic chemistry, and can also be manufactured in accordance with the method described in Japanese Patent Laying-Open No. 1-115902. In addition, a product commercially available from Sigma and the like can also be employed as "hyaluronic acid or a salt thereof' in the present invention, and for example, "sodium hyaluronate" is commercially available from Sigma (catalogue No.: H5388).

Though the present ophthalmic solution can also contain an active ingredient other than "hyaluronic acid or a salt thereof," it preferably contains "hyaluronic acid or salt thereof" as a sole active ingredient.

A concentration of "hyaluronic acid or a salt thereof' in the present ophthalmic solution is from 0.03 to 0.5% (w/v), preferably from 0.1 to 0.3% (w/v), and further preferably 0.1% (w/v) or 0.3% (w/v).

The aforementioned "concentration of hyaluronic acid or a salt thereof" means both of a concentration of "hyaluronic acid (free form)" and a concentration of "salt of hyaluronic acid." For example, "0.1 % (w/v) hyaluronic acid or a salt thereof" means both of a case where a concentration of "hyaluronic acid (free form)" in the ophthalmic solution is 0.1% (w/v) and a case where a concentration of "salt of hyaluronic acid" is 0.1 % (w/v).

A concentration of "propylene glycol" in the present ophthalmic solution is from 0.1 to 1.0% (w/v) and preferably from 0.25 to 0.75% (w/v).

Benzalkonium chloride is a compound shown in a general formula (3) below: 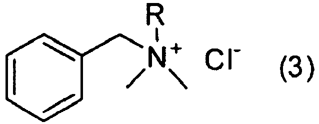 [where R represents an alkyl group having a carbon number from 8 to 18].

Preferred "benzalkonium chloride" in the present invention is benzalkonium chloride in which R in the general formula (3) above represents "C₁₂H₂₅" (hereinafter also referred to as "benzalkonium chloride (C12)").

A concentration of "benzalkonium chloride" in the present ophthalmic solution is from 0.001 to 0.002% (w/v) and preferably from 0.001 to 0.0015% (w/v).

"Comprising benzalkonium chloride as a sole preservative" in the present invention means that the present ophthalmic solution comprises benzalkonium chloride, while it comprises none of benzethonium chloride, chlorhexidine gluconate, parabens, sorbic acid and a salt thereof, chlorobutanol, boric acid, borax, and chlorite.

"Comprising an ionic tonicity agent at such a concentration that an osmotic pressure ratio of the ophthalmic solution becomes from 0.9 to 1.1" in the present invention means that an osmotic pressure ratio of the present ophthalmic solution is adjusted within a range from "0.9 to 1.1" by further adding to the present ophthalmic solution, an ionic tonicity agent in addition to propylene glycol or the like. It is noted that an osmotic pressure ratio of the present ophthalmic solution can readily be measured with the use of an automatic osmolarity analyzer.

The ionic tonicity agent used in the present invention refers to an ionic tonicity agent of sodium chloride, potassium chloride, calcium chloride, magnesium chloride, or the like.

The present ophthalmic solution preferably comprises a buffer and a pH adjuster in such an amount that pH of the ophthalmic solution becomes from 6.0 to 7.0. "Comprising a buffer and a pH adjuster in such an amount that pH of the ophthalmic solution becomes from 6.0 to 7.0" in the present invention means that pH of the present ophthalmic solution is adjusted within a range from "6.0 to 7.0" by adding a buffer and a pH adjuster to the present ophthalmic solution, and an amount of addition (concentration) of a buffer and a pH adjuster is not particularly limited so long as pH of the present ophthalmic solution can be adjusted within that range.

Specific examples of the "buffer" in the present invention include sodium phosphate, dibasic sodium phosphate, disodium phosphate, sodium acetate, epsilon-amino caproic acid, and the like, and epsilon-amino caproic acid is particularly preferred. It is noted that boric acid and borax are not included in the "buffer" in the present invention.

A "pH adjuster" in the present invention is not particularly limited so long as it can adjust pH of the present ophthalmic solution, and specific examples include diluted hydrochloric acid, sodium hydroxide, and the like.

The present ophthalmic solution is substantially equal in kinematic viscosity to the conventional sodium hyaluronate ophthalmic solution. For example, in a case where a concentration of hyaluronic acid or a salt thereof in the present ophthalmic solution is 0.1 % (w/v), kinematic viscosity thereof is within the range from "3.0 to 4.0 mm²/s" described in the Hyalein® ophthalmic solution 0.1% or Hyalein® mini ophthalmic solution 0.1% medical supply interview form. In addition, in a case where a concentration of hyaluronic acid or a salt thereof in the present ophthalmic solution is 0.3% (w/v), kinematic viscosity thereof is within the range from "17 to 30 mm²/s" described in the Hyalein® ophthalmic solution 0.3% or Hyalein® mini ophthalmic solution 0.3% medical supply interview form.

The present ophthalmic solution preferably comprises an edetate at a concentration from 0.001 to 0.1 % (w/v). Here, "edetate" in the present invention means salt of edetic acid (ethylenediaminetetraacetic acid) such as monosodium edetate, disodium edetate, trisodium edetate, and tetrasodium edetate, and a hydrate thereof. Disodium edetate dihydrate (hereinafter also referred to as "sodium edetate hydrate") is preferred as "edetate" in the present invention.

Though a pharmaceutically acceptable additive can be added to the present ophthalmic solution as necessary, the present ophthalmic solution preferably does not contain an additive which is not specified to be contained and influences an effect of the present ophthalmic solution, and further preferably, it does not contain an additive not specified to be contained.

The present invention provides as a preferred embodiment of the present ophthalmic solution described above, an aqueous ophthalmic solution consisting essentially of hyaluronic acid or a salt thereof at a concentration from 0.03 to 0.5% (w/v), propylene glycol at a concentration from 0.1 to 1.0% (w/v), benzalkonium chloride at a concentration from 0.001 to 0.002% (w/v), an ionic tonicity agent at such a concentration that an osmotic pressure ratio of the ophthalmic solution becomes from 0.9 to 1.1, a buffer and a pH adjuster in such an amount that pH of the ophthalmic solution becomes from 6.0 to 7.0, and an edetate at a concentration from 0.001 to 0.1 % (w/v). In the aqueous ophthalmic solution in the present embodiment as well, preferably, a concentration of hyaluronic acid or a salt thereof is from 0.1 to 0.3% (w/v), a concentration of propylene glycol is from 0.25 to 0.75% (w/v), and a concentration of benzalkonium chloride is from 0.001 to 0.0015% (w/v).

"To improve preservative effectiveness" in the present method means that a sodium hyaluronate ophthalmic solution comprising benzalkonium chloride at a concentration not higher than 0.002% (w/v) is improved to pass the defmed preservatives-effectiveness test (for example, the preservatives-effectiveness tests under Japanese Pharmacopoeia, the 15th Edition) without increasing an amount of addition of a preservative.

"Preventing drip" in the present method means that, in a case where an eye drop container is filled with a sodium hyaluronate ophthalmic solution and thereafter an instillation operation is performed, frequency of occurrence of such a phenomenon that the ophthalmic solution runs down from a tip end of the eye drop container along the outside (drip) is lowered as compared with a sodium hyaluronate ophthalmic solution not containing propylene glycol.

In the step (a) of the present method, an ingredient other than sodium hyaluronate, benzalkonium chloride, propylene glycol, and an ionic tonicity agent can also be mixed, and in particular, an edetate in such an amount that a concentration in the sodium hyaluronate ophthalmic solution becomes from 0.001 to 0.1 % (w/v) is preferably mixed.

The step (b) of the present method means adding a buffer and a pH adjuster to a sodium hyaluronate ophthalmic solution to thereby adjust pH within the range from "6.0 to 7.0". So long as pH of the sodium hyaluronate ophthalmic solution can be adjusted within that range, an amount of addition (concentration) of a buffer and a pH adjuster is not particularly limited.

An eye drop container filled with the sodium hyaluronate ophthalmic solution in the step (c) of the present method is not particularly limited so long as it is normally used as an eye drop container, and an eye drop container made of polyethylene is particularly preferred.

The present method can also comprise step(s) other than steps (a), (b), and (c).

Though results of preservatives-effectiveness tests, kinematic viscosity measurement tests, drip check tests, and evaluation test of protective effect on corneal epithelial cell, as well as formulation examples are shown below, these examples are for better understanding of the present invention and do not intend to limit the scope of the present invention.

### Examples

### [Preservatives-Effectiveness Tests]

Preservatives-effectiveness tests were conducted to check influence by propylene glycol on preservative effectiveness of a hyaluronic acid ophthalmic solution.

### (Preparation of Sample)

### <Comparative Formulations 1, 2>

Here, 0.3 g of sodium hyaluronate, 0.7 g of sodium chloride, 0.15 g of potassium chloride, 0.2 g of epsilon-amino caproic acid, 0.01 g of sodium edetate hydrate, and 0.0025 g of benzalkonium chloride (C12) were dissolved in water to obtain a 100 mL product, followed by addition thereto of diluted hydrochloric acid and/or sodium hydroxide. A resultant product having pH of 6.0 was employed as Comparative Formulation 1 and a resultant product having pH of 7.0 was employed as Comparative Formulation 2.

### <Comparative Formulations 3, 4>

Comparative Formulations 3, 4 were prepared as in Comparative Formulations 1 and 2 above, except that an amount of addition of benzalkonium chloride (C12) was set to 0.002 g.

### <Formulation 1>

Here, 0.3 g of sodium hyaluronate, 0.7 g of sodium chloride, 0.25 g of propylene glycol, 0.2 g of epsilon-amino caproic acid, 0.01 g of sodium edetate hydrate, and 0.001 g of benzalkonium chloride (C12) were dissolved in water to obtain a 100 mL product, followed by addition thereto of diluted hydrochloric acid and/or sodium hydroxide. A resultant product had pH of 6.0.

### <Formulation 2>

Formulation 2 was prepared as in Formulation 1 above except that an amount of addition of sodium hyaluronate was set to 0.1 g and an amount of addition of benzalkonium chloride (C12) was set to 0.0012 g.

### (Test Method)

Preservatives-effectiveness tests were conducted in conformity with Preservatives-Effectiveness Tests defined in Japanese Pharmacopoeia, the 15th edition (hereinafter also simply referred to as "Japanese Pharmacopoeia"). In the present test, Escherichia Coli (E. coli), Pseudomonas aeruginosa (P. aeruginosa), Staphylococcus aureus (S. aureus), Candida albicans (C. albicans), and Aspergillus niger (A. niger) were employed as test strains.

### (Test Results)

Table 1 shows test results. In Table 1, "N.D." indicates that no microorganism was detected.

**Table 1**

| Ingredient | | Comparative Formulation 1 | Comparative Formulation 2 | Comparative Formulation 3 | Comparative Formulation 4 | Formulation 1 | Formulation 2 |
|---|---|---|---|---|---|---|---|
| Sodium Hyaluronate | | 0.3% | 0.3% | 0.3% | 0.3% | 0.3% | 0.1% |
| Sodium Chloride | | 0.7% | 0.7% | 0.7% | 0.7% | 0.7% | 0.7% |
| Potassium Chloride | | 0.15% | 0.15% | 0.15% | 0.15% | - | - |
| Propylene Glycol | | - | - | - | - | 0.25% | 0.25% |
| Epsilon-Amino Caproic Acid | | 0.2% | 0.2% | 0.2% | 0.2% | 0.2% | 0.2% |
| Sodium Edetate Hydrate | | 0.01% | 0.01% | 0.01% | 0.01% | 0.01% | 0.01% |
| Benzalkonium Chloride (C12) | | 0.0025% | 0.0025% | 0.002% | 0.002% | 0.001% | 0.0012% |
| Diluted Hydrochloric Acid/Sodium Hydroxide | | q.s. | q.s. | q.s. | q.s. | q.s. | q.s. |
| Purified Water | | q.s. | q.s. | q.s. | q.s. | q.s. | q.s. |
| pH | | 6.0 | 7.0 | 6.0 | 7.0 | 6.0 | 6.0 |

| Results of Preservatives-Effectiveness Tests : Log Reduction | | | | | | | |
|---|---|---|---|---|---|---|---|
| *E. Coli* | 1w | 4.3 | 4.5 | 2.2 | 2.3 | 4.1 | 6.1 |
| | 2w | N.D. | N.D. | 6.1 | N.D. | N.D. | N.D. |
| | 3w | N.D. | N.D. | N.D. | N.D. | N.D. | N.D. |
| | 4w | N.D. | N.D. | N.D. | N.D. | N.D. | N.D. |
| *P. aeruginosa* | 1w | N.D. | N.D. | 2.2 | 5.3 | N.D. | 2.4 |
| | 2w | N.D. | N.D. | 2.5 | 5.6 | N.D. | 4.4 |
| | 3w | N.D. | N.D. | 2.6 | N.D. | N.D. | 4.7 |
| | 4w | N.D. | N.D. | 3.1 | N.D. | N.D. | 5.5 |
| *S*. *aureus* | 1w | 4.8 | 5.4 | 3.0 | 5.1 | 2.2 | 4.3 |
| | 2w | N.D. | N.D. | N.D. | N.D. | N.D. | N.D. |
| | 3w | N.D. | N.D. | N.D. | N.D. | N.D. | N.D. |
| | 4w | N.D. | N.D. | N.D. | N.D. | N.D. | N.D. |
| *C. albicans* | 1w | 0.5 | 0.6 | 0.5 | 1.1 | 0.5 | 0.5 |
| | 2w | 1.5 | 1.9 | 0.9 | 1.8 | 1.2 | 2.4 |
| | 3w | 2.4 | 1.8 | 1.6 | 3.1 | 3.2 | 3.9 |
| | 4w | 3.2 | 5.8 | 2.3 | 4.3 | 4.0 | 4.9 |
| *A. niger* | 1w | 1.6 | 1.5 | 1.0 | 1.2 | -0.2 | -0.2 |
| | 2w | 3.4 | 3.2 | 1.9 | 3.2 | 0.0 | 0.4 |
| | 3w | 4.2 | 4.4 | 2.4 | 3.9 | 0.1 | 0.7 |
| | 4w | 5.3 | N.D. | 3.0 | 4.7 | 0.1 | 0.9 |
| Determination (Based on Japanese Pharmacopoeia) | | Pass | Pass | Fail | Pass | Pass | Pass |

### (Discussion)

As shown in Table 1, it was clarified that the sodium hyaluronate ophthalmic solution satisfied the criteria defined in "Japanese Pharmacopoeia General Information Preservatives-Effectiveness Tests Category IA" within the pH range allowable in the presence of 0.0025% (w/v) benzalkonium chloride (pH 6.0 and 7.0), however, it did not satisfy the criteria at pH 6.0 in the presence of 0.002% (w/v) benzalkonium chloride and did not have sufficient preservative effectiveness. Then, preservative effectiveness at pH 6.0 at which preservative effectiveness was weaker was evaluated in the presence of 0.25% (w/v) propylene glycol. Then, it was clarified that the 0.1% (w/v) sodium hyaluronate ophthalmic solution satisfied the criteria of "Japanese Pharmacopoeia General Information Preservatives-Effectiveness Tests Category IA" at a concentration of benzalkonium chloride of 0.0012% (w/v) and the 0.3% (w/v) sodium hyaluronate ophthalmic solution satisfied the criteria also at a concentration of benzalkonium chloride of 0.001% (w/v) and that both of them had sufficient preservative effectiveness. From the foregoing, it was shown that, by adding propylene glycol, the hyaluronic acid ophthalmic solution achieved sufficient preservative effectiveness even though the concentration of benzalkonium chloride was set to 0.002% (w/v) or lower.

### [Kinematic Viscosity Measurement Test]

Kinematic viscosity measurement tests were conducted in order to check kinematic viscosity of a hyaluronic acid ophthalmic solution in which propylene glycol had been blended in a case that osmotic pressure ratio thereof was adjusted from 0.9 to 1.1.

### (Preparation of Sample)

### <Comparative Formulation>

Here, 0.3 g of sodium hyaluronate, 0.2 g of epsilon-amino caproic acid, 0.8 g of sodium chloride, 0.0015 g of benzalkonium chloride (C12), and 0.01 g of sodium edetate hydrate were dissolved in water to obtain a 100 mL product, followed by addition thereto of diluted hydrochloric acid and/or sodium hydroxide. A resultant product having pH of 6.5 and osmotic pressure ratio of 1.0 was employed as a Comparative Formulation.

### <Formulations 1 to 5>

Formulations 1 to 5 were prepared similarly to Comparative Formulation above except that an amount of addition of sodium chloride and propylene glycol was varied as appropriate to adjust osmotic pressure ratio to 1.0 (see Table 2).

### (Test Method)

According to page 4 of the Hyalein interview form, allowable ranges of osmotic pressure ratio and kinematic viscosity of the 0.3% (w/v) sodium hyaluronate ophthalmic solution are "from 0.9 to 1.1" and "from 17 to 30 mm²/s," respectively. Then, osmotic pressure ratio of the sodium hyaluronate ophthalmic solution was adjusted to "0.9 to 1.1" with the use of propylene glycol and an ionic tonicity agent, and thereafter kinematic viscosity of the osmotic pressure ratio was measured. It is noted that, in the kinematic viscosity measurement test, kinematic viscosity at a measurement temperature of 30°C was measured in accordance with "Japanese Pharmacopoeia General Tests, Viscosity Determination Method I Viscosity Measurement by Capillary Tube." In addition, osmotic pressure ratio was measured with the use of an automatic osmolarity analyzer (manufactured by Arkray, Inc.) in accordance with "Japanese Pharmacopoeia General Tests Osmolarity Determination."

### (Results)

Table 2 shows results.

**Table 2**

| Blended Ingredient | Comparative Formulation | Formulation 1 | Formulation 2 | Formulation 3 | Formulation 4 | Formulation 5 |
|---|---|---|---|---|---|---|
| Sodium Hyaluronate | 0.3% | 0.3% | 0.3% | 0.3% | 0.3% | 0.3% |
| Epsilon-Amino Caproic Acid | 0.2% | 0.2% | 0.2% | 0.2% | 0.2% | 0.2% |
| Sodium Chloride | 0.8% | 0.7% | 0.6% | 0.5% | 0.4% | 0.3% |
| Propylene Glycol | - | 0.25% | 0.5% | 0.75% | 1% | 1.25% |
| Benzalkonium Chloride (C12) | 0.0015% | 0.0015% | 0.0015% | 0.0015% | 0.0015% | 0.0015% |
| Sodium Edetate Hydrate | 0.01% | 0.01% | 0.01% | 0.01% | 0.01% | 0.01% |
| Diluted Hydrochloric Acid/Sodium Hydroxide | q.s. | q.s. | q.s. | q.s. | q.s. | q.s. |
| Purified Water | q.s. | q.s. | q.s. | q.s. | q.s. | q.s. |
| pH | 6.5 | 6.5 | 6.5 | 6.5 | 6.5 | 6.5 |
| Osmotic pressure ratio | 1.0 | 1.0 | 1.0 | 1.0 | 1.0 | 1.0 |
| Kinematic Viscosity (mm²/s) | 25 | 26 | 27 | 28 | 30 | 34 |

### (Discussion)

As is clear from Table 2, it was shown that, with a concentration of blended propylene glycol being 1% (w/v) or lower, kinematic viscosity of the 0.3% (w/v) sodium hyaluronate ophthalmic solution was within the allowable range (17 to 30 mm²/s), while kinematic viscosity exceeded the allowable range of kinematic viscosity at a concentration equal to or higher than that. In addition, it is estimated from the results above that, with a concentration of blended propylene glycol being 1% (w/v) or lower, kinematic viscosity of the 0.1% (w/v) sodium hyaluronate ophthalmic solution will also be within the allowable range (3.0 to 4.0 mm²/s). From the foregoing, it was shown that, in blending propylene glycol in the hyaluronic acid ophthalmic solution, a concentration of blended propylene glycol should be 1.0% (w/v) or lower.

### [Drip Check Test]

A drip check test was conducted in order to check influence by propylene glycol on drip of a hyaluronic acid ophthalmic solution.

### (Preparation of Reagent)

### • Propylene Glycol Free Formulation

Commercially available "Hyalein® ophthalmic solution 0.1%" was employed.

### • Propylene Glycol-Containing Formulation

Sodium hyaluronate (0.1 g), a buffer (epsilon-amino caproic acid), sodium chloride, propylene glycol, benzalkonium chloride (C12), and sodium edetate hydrate were dissolved in water to obtain a 100 mL product (osmotic pressure ratio: 0.9 to 1.1), followed by addition thereto of diluted hydrochloric acid and/or sodium hydroxide. A resultant product had pH of 6.5.

### (Test Method)

An eye drop container made of polyethylene was filled with each of the propylene glycol free formulation or the propylene glycol-containing formulation of 5mL, and 5 healthy subjects performed 6 times an operation the same as the operation performed at the time of ocular instillation. For each operation, whether or not drip (a phenomenon that the ophthalmic solution runs down from the tip end of the eye drop container along the outside) occurred was evaluated.

### (Results)

Table 3 shows results.

**Table 3**

| The Number of Times of Measurement | Propylene Glycol Free Formulation | | Propylene Glycol-Containing Formulation | |
|---|---|---|---|---|
| | Drip | Without Drip | Drip | Without Drip |
| 1 | 1 | 4 | 0 | 5 |
| 2 | 0 | 5 | 0 | 5 |
| 3 | 1 | 4 | 0 | 5 |
| 4 | 0 | 5 | 0 | 5 |
| 5 | 0 | 5 | 0 | 5 |
| 6 | 0 | 5 | 0 | 5 |
| Total | 2 | 28 | 0 | 30 |

### (Discussion)

As is clear from Table 3, with the propylene glycol free formulation, there were some cases of drip, whereas with the propylene glycol-containing formulation, no drip was observed. From the foregoing, it was shown that the present ophthalmic solution exhibited improvement in drip as compared with the conventional hyaluronic acid ophthalmic solution in which no propylene glycol had been blended.

### [Evaluation Test of Protective Effect on Corneal Epithelial Cell]

Evaluation test of protective effect on corneal epithelial cell was conducted in order to study influence by a propylene glycol-containing hyaluronic acid ophthalmic solution on corneal epithelial cell damage due to drying load.

### (Test Method)

SV40 immortalized human corneal epithelial cells: (HCE-T: RIKEN BioResource Center, Japan" Cell No.: RCB2280) were seeded to a 96-well plate (1×10⁴ cells/well) and cultured for one day in an SHEM culture medium. On the next day, the culture medium was replaced with a D-MEM/F12 culture medium containing 0.25% (w/v) propylene glycol, 0.03% (w/v) sodium hyaluronate, or 0.25% (w/v) propylene glycol and 0.03% (w/v) sodium hyaluronate, and thereafter the corneal epithelial cells were cultured for one hour (hereinafter, each also referred to as a "propylene glycol alone group," a "hyaluronic acid alone group," or a "propylene glycol/hyaluronic acid combination group"). It is noted that a group that a culture medium was replaced with a D-MEM/F12 culture medium not containing a tested substance and thereafter the corneal epithelial cells were cultured for one hour was defined as a "vehicle group". After culture, a culture medium of each group was replaced with a D-MEM/F12 culture medium not containing a tested substance and then cells were dried for 7.5 minutes. After drying load, cell viability was measured using Cell Proliferation Assay Kit (manufactured by Promega, catalogue No.: G3580) (corresponding to absorbance of 490 nm).

### (Results)

Fig. 1 shows test results.

### (Discussion)

As is clear from Fig. 1, decrease in cell viability of corneal epithelial cells due to drying load (see the vehicle group) was not sufficiently suppressed by treatment of propylene glycol alone or hyaluronic acid alone (see the propylene glycol alone group, the hyaluronic acid alone group). On the other hand, it was confirmed that, surprisingly, decrease in cell viability was significantly suppressed in the propylene glycol/sodium hyaluronate combination group. From the foregoing results, the propylene glycol-containing hyaluronic acid ophthalmic solution is considered to have an effect to protect corneal epithelial cells against drying.

### [Formulation Example]

Though a drug according to the present invention will be described further specifically with reference to formulation examples, the present invention is not limited only to these formulation examples.

### (Formulation Example 1)

In 100 ml of an ophthalmic solution (0.1 % (w/v))

| | |
|---|---|
| sodium hyaluronate | 0.1 g |
| epsilon-amino-caproic acid | 0.2 g |
| sodium chloride | 0.6 g |
| propylene glycol | 0.5 g |
| benzalkonium chloride (C12) | 0.001 to 0.002 g |
| sodium edetate hydrate | 0.001 to 0.1 g |
| diluted hydrochloric acid | q.s. |
| sodium hydroxide | q.s. |
| sterile purified water | q.s. |

The 0.1 % (w/v) sodium hyaluronate ophthalmic solution having pH from 6.0 to 7.0 and osmotic pressure ratio from 0.9 to 1.1 can be prepared by adding sodium hyaluronate and the above-mentioned ingredients other than that to sterile purified water and sufficiently mixing those.

### (Formulation Example 2)

In 100 ml of an ophthalmic solution (0.3% (w/v))

| | |
|---|---|
| sodium hyaluronate | 0.3 g |
| epsilon-amino-caproic acid | 0.2 g |
| sodium chloride | 0.5 g |
| propylene glycol | 0.75 g |
| benzalkonium chloride (C12) | 0.001 to 0.002 g |
| sodium edetate hydrate | 0.001 to 0.1 g |
| diluted hydrochloric acid | q.s. |
| sodium hydroxide | q.s. |
| sterile purified water | q.s. |

The 0.3% (w/v) sodium hyaluronate ophthalmic solution having pH from 6.0 to 7.0 and osmotic pressure ratio from 0.9 to 1.1 can be prepared by adding sodium hyaluronate and the above-mentioned ingredients other than that to sterile purified water and sufficiently mixing those.

### INDUSTRIAL APPLICABILITY

The hyaluronic acid ophthalmic solution according to the present invention achieves sufficient preservative effectiveness by containing propylene glycol despite the fact that a concentration of benzalkonium chloride in the ophthalmic solution is equal to or lower than 0.002% (w/v), while it has osmotic pressure ratio and kinematic viscosity allowed as the hyaluronic acid ophthalmic solution. In addition, the hyaluronic acid ophthalmic solution according to the present invention has such an effect of less likeliness of drip and protection of corneal epithelial cells against drying, and hence it is expected to serve as an ophthalmic solution capable of more effectively treating dry eye.

## Claims

1. An aqueous ophthalmic solution containing hyaluronic acid or a salt thereof at a concentration from 0.03 to 0.5% (w/v), and propylene glycol at a concentration from 0.1 to 1.0% (w/v), comprising:
benzalkonium chloride at a concentration from 0.001 to 0.002% (w/v) as a sole preservative; and
an ionic tonicity agent at such a concentration that an osmotic pressure ratio of the ophthalmic solution becomes from 0.9 to 1.1.

2. The ophthalmic solution according to claim 1, comprising a buffer and a pH adjuster in such an amount that pH of the ophthalmic solution becomes from 6.0 to 7.0.

3. The ophthalmic solution according to claim 1 or 2, comprising an edetate at a concentration from 0.001 to 0.1 % (w/v).

4. The ophthalmic solution according to claim 1, wherein
a concentration of hyaluronic acid or the salt thereof is from 0.1 to 0.3% (w/v).

5. The ophthalmic solution according to claim 1, wherein
a concentration of propylene glycol is from 0.25 to 0.75% (w/v).

6. The ophthalmic solution according to claim 1, wherein
a concentration of benzalkonium chloride is from 0.001 to 0.0015% (w/v).

7. An aqueous ophthalmic solution, consisting essentially of:
hyaluronic acid or a salt thereof at a concentration from 0.03 to 0.5% (w/v);
propylene glycol at a concentration from 0.1 to 1.0% (w/v);
benzalkonium chloride at a concentration from 0.001 to 0.002% (w/v);
an ionic tonicity agent at such a concentration that an osmotic pressure ratio of the ophthalmic solution becomes from 0.9 to 1.1;
a buffer and a pH adjuster in such an amount that pH of the ophthalmic solution becomes from 6.0 to 7.0; and
an edetate at a concentration from 0.001 to 0.1 % (w/v).

8. The ophthalmic solution according to claim 7, wherein
a concentration of hyaluronic acid or the salt thereof is from 0.1 to 0.3% (w/v),
a concentration of propylene glycol is from 0.25 to 0.75% (w/v), and
a concentration of benzalkonium chloride is from 0.001 to 0.0015% (w/v).

9. A method of improving preservative effectiveness of an aqueous ophthalmic solution containing sodium hyaluronate at a concentration of 0.1 % (w/v) and benzalkonium chloride as a sole preservative, setting kinematic viscosity of the ophthalmic solution to 3.0 to 4.0 mm²/s, and preventing drip of the ophthalmic solution, comprising:
a step (a) of mixing sodium hyaluronate in such an amount that a concentration in the ophthalmic solution becomes 0.1 % (w/v), benzalkonium chloride in such an amount that a concentration in the ophthalmic solution becomes from 0.001 to 0.002% (w/v), propylene glycol in such an amount that a concentration in the ophthalmic solution becomes from 0.1 to 1.0% (w/v), and an ionic tonicity agent in such an amount that an osmotic pressure ratio of the ophthalmic solution becomes from 0.9 to 1.1;
a step (b) of adjusting pH of the ophthalmic solution to 6.0 to 7.0; and
a step (c) of filling an eye drop container with the ophthalmic solution.

10. A method of improving preservative effectiveness of an aqueous ophthalmic solution containing sodium hyaluronate at a concentration of 0.3% (w/v) and benzalkonium chloride as a sole preservative, setting kinematic viscosity of the ophthalmic solution to 17 to 30 mm²/s, and preventing drip of the ophthalmic solution, comprising:
a step (a) of mixing sodium hyaluronate in such an amount that a concentration in the ophthalmic solution becomes 0.3% (w/v), benzalkonium chloride in such an amount that a concentration in ophthalmic solution becomes from 0.001 to 0.002% (w/v), propylene glycol in such an amount that a concentration in the ophthalmic solution becomes from 0.1 to 1.0% (w/v), and an ionic tonicity agent in such an amount that an osmotic pressure ratio of the ophthalmic solution to 0.9 to 1.1;
a step (b) of adjusting pH of the ophthalmic solution to 6.0 to 7.0; and
a step (c) of filling an eye drop container with the ophthalmic solution.

11. The method according to claim 9 or 10, wherein
in the step (a), an edetate in such an amount that a concentration in the ophthalmic solution becomes from 0.001 to 0.1 % (w/v) is further mixed.

12. The method according to claim 9 or 10, wherein
in the step (a), propylene glycol in such an amount that a concentration in the ophthalmic solution becomes from 0.25 to 0.75% (w/v) is mixed.

13. The method according to claim 9 or 10, wherein
in the step (a), benzalkonium chloride in such an amount that a concentration in the ophthalmic solution becomes from 0.001 to 0.0015% (w/v) is mixed.
